# DEMANDE DE BREVET EUROPEEN

(11) **EP 1 992 336 A2**
(43) Date de publication de la demande: **19.11.2008**
(21) Numéro de dépôt: 08163660.7
(22) Date de dépôt: 06.08.2003
(51) Int. Cl.: A61K 9/16, A61K 9/50

(54) **Forme galénique pour la délivrance colique de principes actifs**

(30) Priorité: 09.08.2002 FR 0210151; 29.10.2002 FR 0213514
(62) Demande divisionnaire de: 03758212.9
(71) Demandeur: DA Volterra, 75008 Paris (FR); CENTRE NATIONAL DE LA RECHERCHE SCIENTIFIQUE C.N.R.S), 75016 Paris (FR)
(72) Inventeur: Bourgeois, Sandrine, 75013, PARIS (FR); Fattal, Elias, 75012, PARIS (FR); Andremont, Antoine, MALAKOFF, 92240 (FR); Couvreur, Patrick, 91140, VILLEBON-SUR-YVETTE (FR)
(74) Mandataire: Portal, Frédéric

(57) **Abrégé**

Formes galéniques multiparticulaires aptes à être utilisées par voie orale et destinées à la délivrance colique de principes actifs choisis dans le groupe comprenant les enzymes capables d'inactiver les macrolides et apparentés, les enzymes capables d'inactiver les macrolides et apparentés, les enzymes capables d'inactiver les quinolones et les β-lactamases.

## Description

La présente invention concerne une forme galénique à délivrance colique, son procédé de préparation et son utilisation en thérapeutique.

Les systèmes de libération spécifique au niveau du colon ont été reconnus comme présentant d'importants avantages thérapeutiques.

Un grand nombre de maladies coliques pourraient effectivement être traitées plus efficacement si le principe actif est libéré localement. C'est le cas, entre autres, de la maladie de Crohn, des colites ulcératives, du cancer colorectal et de la constipation.

La libération colique pourrait aussi être intéressante quand, du point de vue thérapeutique, un retard à l'absorption est nécessaire, notamment dans le traitement de pathologies comme l'asthme nocturne ou l'angor (Kinget R. et al. (1998), Colonic Drug Targeting, Journal of Drug Targeting, 6, 129).

L'administration de principes actifs polypeptidiques se fait essentiellement par voie parentérale, qui est douloureuse et à l'origine d'une mauvaise observance des traitements. Depuis quelques années, il y a donc un intérêt pour l'utilisation du colon comme site d'absorption des principes actifs peptidiques (analgésiques, contraceptifs, vaccins, insuline...). L'absorption des peptides au niveau du colon semble effectivement meilleure qu'au niveau des autres sites du tube digestif grâce, notamment, à une activité protéolytique nettement plus faible qu'au niveau de l'intestin grêle et à l'absence d'une activité peptidasique associée à la membrane des cellules épithéliales coliques.

Lors de l'administration d'antibiotiques par la bouche, ceux-ci traversent l'estomac puis sont absorbés au niveau de l'intestin grêle pour diffuser dans l'ensemble de l'organisme et traiter le foyer infectieux pour lequel ils ont été administrés. Toutefois, une fraction des antibiotiques ingérés (dont l'importance varie avec les caractéristiques propres de chaque type d'antibiotiques) n'est pas absorbée et continue sa progression jusqu'au colon avant d'être éliminée dans les selles. Ces antibiotiques résiduels sont rejoints, au niveau de l'intestin grêle, par une fraction des antibiotiques absorbés mais qui sont ré-excrétés dans le tube digestif par l'intermédiaire de l'élimination biliaire. Cette fraction est d'importance variable en fonction du métabolisme et des voies d'élimination de chaque antibiotique. Enfin, pour certains antibiotiques, une fraction de la dose absorbée est éliminée directement par la muqueuse intestinale dans la lumière du tube digestif. Ainsi, que les antibiotiques aient été administrés par voie orale ou par voie parentérale, une fraction résiduelle active se retrouve généralement au niveau du colon. Cela est vrai, à des degrés divers, pour la grande majorité des familles d'antibiotiques utilisés en thérapeutique, la seule exception notable étant la famille des amino-glycosides pour lesquels l'excrétion intestinale est négligeable. Pour les autres antibiotiques, l'excrétion intestinale d'une activité antibiotique résiduelle va avoir différentes conséquences, toutes délétères. En effet, il existe au niveau du colon un écosystème bactérien complexe (plusieurs centaines d'espèces bactériennes différentes) et très dense (plus de 10¹¹ bactéries par gramme de contenu colique) qui va être affecté par l'arrivée des résidus actifs d'antibiotiques. On pourra observer:
1) un déséquilibre de flore qui serait la cause principale des diarrhées banales suivant parfois la prise d'antibiotiques (Bartlett J.G. (2002) Clinical practice. Antibiotic associated diarrhea, New England Journal of Medicine, 346, 334). Bien que ces diarrhées ne soient en règle générale pas graves et cèdent rapidement, soit spontanément, soit à l'arrêt du traitement, elles sont toutefois mal ressenties par les patients et ajoutent à l'inconfort de la maladie de base pour laquelle l'antibiotique a été prescrit;
2) une perturbation des fonctions de résistance à la colonisation par des bactéries exogènes (ou "effet barrière") avec possibilités de risque accru d'infection, par exemple, intoxication alimentaire à salmonelle (Holmberg S.D. et al. (1984) Drug resistant Salmonella from animals fed antimicrobials, New England Journal of Medicine, 311, 617) ;
3) la sélection de micro-organismes résistants à l'antibiotique. Ces derniers peuvent être de divers types:
   a) il peut d'abord s'agir de bactéries pathogènes comme par exemple, *Clostridium difficile,* espèce capable de sécréter des toxines causant de redoutables colites dites pseudomembraneuses (Bartlett J.G. (1997) Clostridium difficile infection: pathophysiology and diagnosis, Seminar in Gastrointestinal Disease, 8, 12);
   b) il peut aussi s'agir de microorganismes relativement peu pathogènes mais dont la multiplication peut amener à une infection de voisinage (Candidose vaginale ou cystite à *Escherichia coli* résistant).
   c) Il peut enfin s'agir de bactéries résistantes commensales non pathogènes mais dont la multiplication et l'élimination fécale va accroître la dissémination dans l'environnement. Or, ces bactéries commensales résistantes peuvent constituer une source importante de mécanismes de résistance pour des espèces pathogènes. Ce risque est actuellement considéré comme majeur en raison du caractère inquiétant de l'évolution vers la multirésistance de nombreuses espèces pathogènes pour l'homme.

De nombreuses stratégies exploitant les divers paramètres physiologiques du tube digestif ont donc été envisagées dans le but de libérer des principes actifs au niveau du colon. Des études ont notamment été réalisées à l'aide de systèmes d'administration basés sur (1) l'utilisation de polymères sensibles aux variations de pH, (2) de formes à libération dépendante du temps, (3) de prodrogues ou encore de polymères dégradables par les bactéries de la flore.

### (1) Les systèmes basés sur les variations de pH.

Le pH au niveau de l'estomac est de l'ordre de 1 à 3 mais il augmente dans l'intestin grêle et le colon pour atteindre des valeurs voisines de 7 (Hovgaard L. et al. (1996) Current Applications of Polysaccharides in Colon Targeting, Critical Reviews in Therapeutic Drug Carrier Systems, 13, 185). Pour qu'un principe actif atteigne le colon, sans subir ces variations de pH, il est possible de l'administrer sous forme de comprimés, de gélules ou de sphéroïdes enrobés avec un polymère pH dépendant, insoluble à pH acide mais soluble à des pH neutres ou alcalins (Kinget *et al.* déjà cité). Les polymères les plus couramment utilisés sont des dérivés de l'acide méthacrylique, les Eudragit^{®} L et S (Ashford M. et al. (1993), An in vivo investigation into the suitability of pH-dependent polymers for colonic targeting, International Journal of Pharmaceutics, 95, 193 et 95, 241; et David A. et al. (1997) Acrylic polymers for colon-specific drug delivery, S.T.P. Pharma Sciences, 7, 546).

Etant donné l'importante variabilité inter- et intra-individuelle des valeurs de pH existant au niveau du tractus gastro-intestinal, les polymères pH dépendants ne représentent pas le meilleur moyen pour obtenir une libération spécifique au niveau du colon (Ashford M. *et al.,* déjà cité).

### (2) Les systèmes basés sur le temps de transit.

La formulation de ces systèmes est telle qu'elle permet une libération des principes actifs après un laps de temps prédéfini. Afin de libérer le principe actif au niveau du colon, ces formes doivent à la fois résister à l'environnement acide de l'estomac et entrer dans une phase silencieuse d'un temps prédéterminé, avant de libérer le principe actif, correspondant au temps de transit de la bouche à l'iléon terminal (Gazzaniga A. et al. (1995) Time-dependent oral delivery systems for colon targeting, S.T.P. Pharma Sciences, 5, 83 et 108, 77; Liu P. et al. (1999) Alginate/Pectin/Poly-L-lysine particulate as a potential controlled release formulation, J. Pharm. Pharmacol., 51, 141; Pozzi F. et al. (1994) The Time Clock system: a new oral dosage form for fast and complete release of drug after predetermined lag time, Journal of Controlled Release, 31, 99).

La Pulsincap^{®} de Scherer fut parmi les premières formulations de ce type (demande internationale WO 90/09168). Elle a l'apparence d'une gélule dont le corps est insoluble dans l'eau. Le principe actif est maintenu dans le corps par un bouchon d'hydrogel placé au niveau de la tête de la gélule hydrosoluble. Le tout est enrobé d'un film gastro-résistant. Après dissolution de la tête au niveau de l'intestin grêle, le bouchon, au contact des liquides digestifs, gonfle. Quand ce dernier atteint un seuil de gonflement critique il est éjecté, permettant ainsi la libération du principe actif. Le temps d'éjection est contrôlé par les propriétés de l'hydrogel composant le bouchon.

Les systèmes basés sur le temps de transit présentent toutefois de nombreux inconvénients (variabilités des temps de vidange gastrique et de transit, phénomènes de rétention au niveau de la valvule iléo-caecale (Kinget R., déjà cité), leur procurant un manque de spécificité et empêchant d'aboutir à une validation de ces derniers comme systèmes de libération spécifique au niveau du colon. Enfin la production à large échelle de ce type de systèmes est difficilement envisageable car elle nécessiterait une adaptation importante et coûteuse des technologies industrielles.

Récemment une nouvelle forme pour le ciblage colique a été développée, la "*Colon*-*targeted Delivery Capsule*" (CTDC) (Ishibashi T. et al. (1998) Design and evaluation of a new capsule-type dosage form for colon-targeted delivery of drugs, International Journal of Pharmaceutics, 168, 31 et 57, 45). La CTDC est un système associant à la fois le facteur pH-dépendant et le facteur temps-dépendant. Il se présente sous la forme d'une gélule classique renfermant le principe actif et un acide organique (acide succinique), recouverte de 3 couches.

### (3) Les systèmes basés sur l'activité enzymatique de la flore microbienne colique.

### 3.1. Les prodrogues_{.}

Les prodrogues ont largement été étudiées pour le ciblage colique de principes actifs divers (anti-inflammatoires non stéroïdiens et stéroïdiens, spasmolytiques...). Ces systèmes sont basés sur la capacité qu'ont les enzymes produites par la flore colique de dégrader les prodrogues afin de libérer la forme active du principe actif.

De nombreuses prodrogues basées sur l'action des azoréductases bactériennes ont notamment été développées dans le but de libérer au niveau du colon, des principes actifs tels que l'acide 5-aminosalicylique (5-ASA) utilisé dans le traitement de pathologies locales comme la maladie de Crohn ou les colites ulcératives (Peppercorn M.A. et al. (1972) The role of intestinal bacteria in the metabolism of salicylazosulfapyridine, The Journal of Pharmacology and Experimental Therapeutics, 181, 555 et 64, 240).

Une autre approche consiste à exploiter les hydrolases bactériennes comme les glycosidases et les polysaccharidases (Friend D.R. (1995) Glycoside prodrugs: novel pharmacotherapy for colonie diseases, S.T.P. Pharma Sciences, 5, 70; Friend D.R. et al. (1984) A colon-specific drug-delivery system based on drug glycosides and the glycosidases of colonic bacteria, Journal of Medicinal Chemistry, 27, 261; Friend D.R. et al. (1985) Drug glycosides: potential prodrugs for colon-specific drug delivery, Journal of Medicinal Chemistry, 28, 51; et Friend D.R. et al. (1992) Drug glycosides in oral colon-specific drug delivery, Journal of Controlled Release, 19, 109). Des prodrogues ont ainsi été développées en couplant, par exemple, des stéroïdes à des sucres (glucose, galactose, cellobiose, dextrane (demande internationale WO 90/09168)), cyclodextrines (Hirayama F. et al. (1996) In vitro evaluation of Biphenylyl Acetic Acid-β-Cyclodextrin conjugates as colon-targeting, prodrugs: drug release behavior in rat biological média, Journal of. Pharmacy and Pharmacology, 48, 27).

### 3.2. L'enrobage par des polymères biodégradables par les enzymes bactériennes.

Dans ce cas, le ciblage colique se fait par enrobage de la forme pharmaceutique avec un polymère spécifiquement dégradé par les enzymes produites par la microflore, en mettant à profit la présence des azoréductases ou des glycosidases bactériennes.

De nombreux polymères renfermant des liaisons azoaromatiques ont été utilisés pour enrober un principe actif. Saffran et al. (Oral insulin in diabetic dogs, Journal of Endocrinology (1991), 131, 267 et A new approach to the oral administration of insulin and other peptide drugs, Science (1986), 233, 1081) ont ainsi décrit la libération d'insuline et de vasopressine dans le colon de rats et de chiens à partir de formes orales enrobées avec des copolymères de styrène et d'hydroxyéthylméthacrylate (HEMA) reliés par des liaisons azoaromatiques. Cet enrobage est dégradé dans le colon par les azoréductases bactériennes entraînant ainsi la libération de la substance active.

L'avantage des azopolymères est qu'ils permettent une très bonne sélectivité colique pour la libération de principes actifs. L'inconvénient lié à leur utilisation est le manque de connaissances sur leur éventuelle toxicité.

Pour éviter cet inconvénient, d'autres études se sont plutôt portées sur l'utilisation de film d'enrobage à base de substances naturelles comme les polysaccharides avec notamment des films d'enrobage à base d'amylose/éthylcellulose (Milojevic S. et al. (1996) Amylose as a coating for drug delivery to the colon: preparation and in vitro evaluation using 5-aminosalicylic acid pellets, Journal of Controlled Release, 38, 75), à base d'un ester de dextrane (Bauer K.H. et al. (1995) Novel pharmaceutical excipients for colon targeting, S.T.P. Pharma Sciences, 5, 54) ou de pectine.

### 3.3. Les matrices biodégradables par les enzymes bactériennes.

Une autre approche de systèmes à libération spécifique au niveau du colon consiste en l'élaboration de matrices par compression d'un mélange de principe actif et de polymères biodégradables comme la chondroïtine sulfate (Rubinstein A. et al. (1992b) Chondroitin sulfate: a potential biodegradable carrier for colon-specific drug delivery, International Journal of Pharmaceutics, 84, 141 et Rubinstein A. et al. (1992a) Colonic drug delivery: enhanced release of Indomethacin from cross-linked chondroitin matrix in rat cecal content, Pharmaceutical Research, 9, 276), la gomme guar (Krishnaiah Y.S.R. et al. (1998) Evaluation of guar gum as a compression coat for drug targeting to colon, International Journal of Pharmaceutics, 171, 137), le chitosan (Tozaki H. et al. (1997) Chitosan capsules for colon-specific drug delivery: improvement of insulin absorption from the rat colon, Journal of Pharmaceutical Sciences, 86, 1016) ou la pectine (Rubinstein A. et al. (1993) In vitro évaluation of calcium pectinate: a potential colon-specific drug delivery carrier, Pharmaceutical Research, 10, 258).

Les systèmes basés sur l'activité enzymatique de la flore microbienne sont probablement ceux présentant le plus de spécificité colique pour la libération des principes actifs. Ils constituent donc une voie d'avenir pour le ciblage colique.

L'intérêt des polysaccharides dans la préparation de systèmes pour administration colique réside dans le fait qu'ils sont d'origine naturelle, faiblement toxiques et spécifiquement dégradées par les enzymes bactériennes de la flore colique.

Ainsi, la pectine est un polysaccharide isolé des parois cellulaires des végétaux supérieurs, largement utilisé dans l'industrie agro-alimentaire (comme gélifiant ou épaississant de confitures, glaces...) et pharmaceutique. Elle est polymoléculaire et polydisperse. Sa composition varie selon la source, les conditions d'extraction et les facteurs environnementaux.

Les pectines sont principalement composées d'enchaînements linéaires d'acides α-1,4-(D) - galacturoniques, parfois entrecoupés par des unités de rhamnose. Les groupements carboxyliques des acides galacturoniques peuvent être partiellement estérifiés pour donner des pectines méthylées. On distingue deux sortes de pectine selon leur degré de méthylation (DM: nombre de groupement méthoxy- pour 100 unités d'acide galacturonique):
- la pectine hautement méthylée (HM: high methoxy) dont le degré de méthylation varie entre 50 et 80%. Elle est peu soluble dans l'eau et forme des gels en milieu acide (pH<3,6) ou en présence de sucres;
- la pectine faiblement méthylée (LM: low methoxy), avec un degré de méthylation allant de 25 a 50%. Plus soluble dans l'eau que la pectine HM, elle donne des gels en présence de cations divalents comme les ions Ca²⁺. En effet, les ions Ca²⁺ forment des "ponts" entre les groupements carboxyles libres des acides galacturoniques. Le réseau ainsi formé a été décrit par Grant *et al.* sous le nom de «egg-box model» (Grant G.T. et al. (1973) Biological interactions between polysaccharides and divalent cations: the egg-box model, FEBS Letters, 32, 195).

Il existe aussi des pectines amidées. Par traitement de la pectine par l'ammoniaque certains groupements carboxylate de méthyle (-COOCH₃) peuvent être transformés en groupements carboxamides (-CONH₂). Cette amidation confère de nouvelles propriétés aux pectines, notamment une meilleure résistance aux variations de pH.

La pectine est dégradée par des enzymes issues de végétaux supérieurs et de divers microorganismes (champignons, bactéries...) parmi lesquels on retrouve les bactéries de la flore colique humaine. Les enzymes produites par la microflore sont composées d'un ensemble de polysaccharidases, de glycosidases et d'estérases.

L'enrobage d'une forme galénique par la pectine s'effectue soit par compression (Ashford M. et al. (1993b), An evaluation of pectin as a carrier for drug targeting to the colon, Journal of Controlled Release, 26, 213), soit par pulvérisation. L'enrobage par compression est généralement réalisé avec de la pectine seule alors que celui par pulvérisation nécessite l'emploi d'un polymère filmogène en plus de la pectine (Milojevic S. et al. (1996) Amylose as a coating for drug delivery to the colon: préparation and in vitro evaluation using 5-aminosalicylic acid pellets, Journal of Controlled Release, 38, 75; Wakerly Z. et al. (1996) Pectin/ethycellulose film coating formulations for colonic drug delivery, Pharmaceutical Research, 13, 1210).

De nombreuses formes matricielles à base de pectine ont également été étudiées. Elles sont généralement constituées soit de pectine pure, soit de son complexe avec les *ions Ca*²⁺, faiblement hydrosoluble, le pectinate de calcium. Une matrice de pectinate de calcium renfermant de l'indométacine a notamment été décrite par Rubinstein et al. (1992a) Colonic drug delivery: enhanced release of Indomethacin from cross-linked chondroitin matrix in rat cecal content, Pharmaceutical Research, 9, 276) montrant une meilleure stabilité du pectinate de calcium que la pectine seule dans les milieux digestifs, tout en restant sensible à l'action des enzymes pectinolytiques.

Les pectines amidées, plus tolérantes aux variations de pH ont aussi été étudiées pour l'élaboration de comprimés matriciels à visée colique (Wakerly Z, et al. (1997) Studies on amidated pectins as potential carriers in colonic drug delivery, Journal of Pharmacy and Parmacologyl. 49, 622).

Aydin et al. ((1996) Preparation and évaluation of pectin beads, International Journal of Pharmaceutics, 137, 133) ont été les premiers à formuler des billes de pectine selon la méthode de gélification ionique de Bodmeier et al. ((1989) Preparation and evaluation of drug-containing chitosan beads, Drug Developpent and Industrial Pharmacy, 15, 1475 et Spherical agglomerates of water-insoluble drugs, Journal of Pharmaceutical Sciences, 78, 964) qui avait mis au point des billes d'alginate et de chitosane. Leur objectif était d'incorporer dans les billes deux principes actifs différents, un cationique (l'aténolol) et un anionique (le piroxicam), afin de caractériser d'éventuelles interactions avec la pectine. Ils ont ainsi montré qu'il était possible de former des billes avec les 2 types de principe actifs et que les conditions opératoires avaient une influence capitale sur les propriétés des billes obtenues.

Sriamornsak s'est servi de billes de pectinate de calcium afin d'établir un système pour la libération spécifique de protéines au niveau du colon, en utilisant la serum albumine bovine (BSA) d'un poids moléculaire de 66400 Da comme protéine modèle (Sriamornsak P. (1998) Investigation on pectin as a carrier for oral delivery of proteins using calcium pectinate gel beads, International Journal of Pharmaceutics, 169, 213 et (1999) Effect of calcium concentration, hardening agent and drying condition on release characteristics of oral proteins from calcium pectinate gel beads, European Journal of Pharmaceutical Sciences, 8, 221). Il a étudié l'influence de différents facteurs de formulation sur les propriétés des billes obtenues comme leur forme, leur taille, le taux d'encapsulation de la BSA et sa cinétique de libération. Sriamornsak a donc démontré que les billes de pectinate de Ca pourraient être employées pour la libération spécifique de protéines au niveau du colon. L'obtention d'un profil de cinétique de libération adéquat dépend principalement du choix de la formulation et des conditions opératoires pour la préparation des billes. Aucune corrélation *in vitro*/*in vivo* des profils de libération des principes actifs encapsulées n'a été établie.

Afin d'augmenter la stabilité des particules le long du tractus digestif et d'éviter toute libération prématurée du principe actif encapsulé, il est possible de renforcer les billes de pectine en les réticulant avec un polymère cationique.

Munjeri et al. ((1997) Hydrogel beads based on amidated pectins for colon-specific drug delivery: the role of chitosan in modifying drug release, Journal of Controlled Release, 46, 273) ont réticulé des billes de pectine amidée avec du chitosane. Ils ont alors montré, en comparant les cinétiques de dissolution de formes réticulées et de formes non réticulées, que le chitosane permettait de minimiser la libération des principes actifs insolubles mais ne modifiait pas significativement la libération des principes actifs hydrosolubles. La perte de principe actif dans des conditions mimant celles de l'estomac et de l'intestin grêle peut donc être limitée par la formation d'un complexe entre le chitosane et la pectine amidée; les billes de pectine réticulées restant toujours sensibles à l'action des enzymes pectinolytiques coliques.

Un autre agent réticulant, la polylysine, a été testé en présence de billes d'alginate/pectine (Liu P. et al. (1999) Alginate/Pectin/Poly-L-lysine particulate as a potential controlled release formulation, J. Pharm. Pharmacol., 51,141). Les billes réticulées par la polylysine semblent libérer moins de principe actif en milieu acide (HCl 0,1N) que les billes non réticulées, sauf en présence de principes actifs très hydrosolubles. Le même type d'effet est retrouvé en milieu alcalin (tampon phosphate pH 7,5) mais il est nettement moins marqué qu'en milieu acide.

La demande de brevet internationale WO 88/07865 suggère d'administrer au niveau du colon des bactéries productrices de β-lactamases afin d'hydrolyser les antibiotiques résiduels. Les micro-organismes utilisés sont des bactéries à métabolisme anaérobie strict, dont la production et la lyophilisation en quantité suffisante pour élaborer un médicament sont difficiles. De plus, elles sont porteuses de gènes de résistance aux antibiotiques encodant pour des β-lactamases engendrant ainsi un risque de dissémination de ces gènes au sein de l'écosystème colique et dans l'environnement.

La demande de brevet internationale WO 93/13795 propose une forme galénique orale contenant des β-lactamases. Elle peut être composée de particules de saccharose de 1 à 2,5 mm de diamètre renfermant les β-lactamases ou des amidases et éventuellement un inhibiteur de la trypsine, lesdites particules étant recouvertes d'un polymère gastrorésistant. Ces particules auraient la possibilité de libérer l'enzyme au niveau des différents segments du tube digestif pour que son activité s'exerce selon les besoins au niveau désiré de l'intestin.

Aucun des exemples ne comporte de données expérimentales montrant que la formulation galénique proposée est effectivement capable de délivrer l'enzyme sous forme active au niveau désiré de l'intestin. De plus, aucune preuve de la capacité de la préparation galénique à hydrolyser effectivement l'antibiotique *in vivo,* ni même *in vitro* dans un milieu reproduisant les caractéristiques du milieu intestinal n'est apportée.

Pour toutes ces raisons, il est hautement souhaitable de disposer d'un système destiné à réduire la quantité des antibiotiques résiduels qui parviennent au colon après antibiothérapie orale ou parentérale, ou capable de délivrer un principe actif directement au niveau du colon.

Ainsi, la présente invention a donc pour objet des formes galéniques multiparticulaires aptes à être utilisées par voie orale et destinées à la délivrance colique de principes actifs.

Au sens de la présente invention, on entend par principe actif une substance ou composition qui est apte à être utilisée en thérapeutique ou en diagnostic et peut être incorporée dans la forme galénique selon l'invention.

Le principe actif peut être un anti-infectieux, par exemple les antibiotique, des composés anti-inflammatoires, anti-histaminiques, anti-cholinergiques, antiviraux, antimitotiques, des peptides, des protéines, des gènes, des oligonucléotides anti-sens, des agents de diagnostic et/ou des agents immunosuppressifs ou des bactéries.

Parmi les principes actifs particulièrement avantageux, on trouve les agents anti-inflammatoires, les agents antitumoraux, les oligonucléotides anti-sens et les enzymes capables d'inactiver les antibiotiques au niveau du colon, notamment les β-lactamases ou les enzymes capables d'inactiver les macrolides et apparentés comme l'érythromycine estérase décrite par Andremont A. et al. ((1985) Plasmid mediated susceptibility to intestinal microbial antagonisms in Escherichia coli Infect. Immun. 49(3), 751) ou capables d'inactiver les quinolones comme ceux décrits par Chen Y et al. ((1997) Microbicidal models of soil metabolisms biotransformations of danofloxacin Journal of Industrial Microbiology and Biotechnology 19, 378).

Les principes actifs peuvent être hydrosolubles ou liposolubles.

Dans un mode avantageux de réalisation de l'invention, les formes galéniques multiparticulaires aptes à être utilisées par voie orale et destinées à la délivrance colique de principes actifs comprennent des billes de pectine présentes sous forme d'un sel cationique renfermant le principe actif, ladite pectine étant réticulée par un polymère cationique.

Dans un mode avantageux de réalisation selon l'invention, le polymère cationique qui permet la réticulation de la pectine est choisi dans le groupe composé de la polyéthylènimine, de la polylysine, du chitosan et de leurs dérivés.

Plus avantageusement, le poids moléculaire de ces polymères cationique, est compris entre 10 000 et 100 000 Daltons, de préférence entre 20 000 et 50 000 Daltons.

Dans un autre mode avantageux de l'invention, le sel cationique de pectine utilisé est le pectinate de calcium.

Au sens de la présente invention, on entend par pectine aussi bien de la pectine méthylée ou non méthylée, amidée ou non amidée.

Les formes galéniques selon l'invention, peuvent être administrées sous toutes formes orales, notamment sous forme de gélules et de capsules.

Ces gélules et ces capsules peuvent être administrées simultanément ou successivement avec d'autres principes actifs, notamment lorsque les gélules ou les capsules contiennent des enzymes capables d'inactiver les antibiotiques, elles peuvent être administrées simultanément ou successivement avec la préparation d'antibiotiques correspondants.

Les principes actifs administrés conjointement aux gélules et capsules contenant les formes galéniques selon l'invention sont administrés oralement ou par toute autre voie.

Les formes galéniques selon l'invention peuvent être préparées par des méthodes connues de l'homme du métier ou par des procédés nouveaux qui font également partie de l'invention.

Ainsi, la présente invention a également pour objet un procédé de préparation des formes galéniques multiparticulaires caractérisé en ce que l'on introduit goutte à goutte une solution aqueuse de pectine contenant le principe actif à une concentration de 0,5 à 5% (v/v) dans une solution de chlorure de calcium pour former les billes de pectinate de calcium, puis on récupère les billes de pectinate de calcium ainsi obtenues et on les introduit dans une solution aqueuse du polymère cationique.

Dans un mode de réalisation avantageux du procédé, la solution de pectine est de 4 à 10% (m/v), de préférence de 4 à 7%, la solution de chlorure de calcium de 2 à 10% (m/v) et la solution de polymère cationique de 0,5 à 2% (m/v), ladite solution de polymère cationique étant de préférence une solution de polyéthylènimine.

Dans un mode encore plus avantageux de réalisation de l'invention, les formes galéniques sont préparées à partir d'une solution de pectine à 6% (m/v), d'une solution de chlorure de calcium à 6% (m/v) et d'une solution de polyéthylènimine à 1% ou à 0,6%.

Les billes sont maintenues dans le chlorure de calcium sous agitation lente pendant 10 min à 1 heure, de préférence pendant 20 min.

L'étape de réticulation par le polymère cationique est réalisée sous agitation lente pendant 15 à 40 min, de préférence pendant 20 min.

Après récupération des billes de pectinate, les billes sont séchées à une température comprise entre 20 et 40°C pendant 30 min à 10 heures, de préférence à 37°C pendant 2 heures.

Le diamètre des particules selon l'invention est compris entre 800 et 1 500 µm, de préférence entre 1 000 et 1 200 µm.

Les rendements d'encapsulation sont compris entre 50 et 90% soit 3-6 UI/billes de β-lactamases, activité exprimée en substrat benzylpénicilline, que la pectine soit amidée ou non.

La stabilité en milieu gastrique est supérieure à 10 heures et est également très bonne en milieu intestinale USP XXIV, puisqu'elle est supérieure à 7 heures (la durée de stabilité des billes de pectine non réticulées n'excède pas 1 heure) et ce quelque soit le type de pectine utilisé.

Les exemples 1 à 7 et les figures 1 à 8 qui suivent illustrent l'invention.

La figure 1 représente l'effet de la réticulation avec différentes concentrations de PEI (0,6 ; 0,7 ; 0,8 ; 0,9 et 1%) sur les temps de désagrégation de billes de pectine amidée, placées dans trois milieux différents: PBS, 0,01 M, pH à 7,4; milieu intestinal au pH de 6,8 + 0,1 UPS XXIV; milieu gastrique au pH de 1,1 USP XXIV.

La figure 2 illustre la structure des billes contenant des β-lactamases à raison de 4,4 UI/bille et réticulées pendant 20 minutes à la PEI à 1% et observées en microscopie électronique à balayage.

La figure 3 illustre la libération des β-lactamases in vitro à partir de billes de pectine amidée réticulées préparées selon l'exemple 1 avec des concentrations en PEI de 0,6 et 0,7% et contenant environ 5 UI/bille, placées en milieu intestinal USP XXIV puis en milieu colique (tampon HEPES pH 6 + enzymes pectinolytiques).

La figure 4 illustre l'évolution de l'activité β-lactamase dans les selles de souris en fonction du temps, après administration orale de billes de pectine réticulées à la PEI préparées selon l'exemple 1 et contenant 4,4 UI/bille.

La figure 5 illustre la structure des billes contenant des β-lactamases à raison de 4,4 UI/bille 30 minutes après administration *in vivo.* Les billes sont alors dans l'estomac. A et B représentant les billes entières et C et D les billes coupées.

La figure 6 illustre la structure des billes contenant des β-lactamases à raison de 4,4 UI/bille 2 heures après administration *in vivo.* Les billes sont alors dans l'intestin grêle. A et B représentant les billes entières et C et D les billes coupées.

La figure 7 illustre la structure des billes contenant des β-lactamases à raison de 4,4 UI/bille 4 heures après administration *in* vivo. Les billes sont alors dans le colon. A et B représentant les billes entières et C et D les billes coupées.

La figure 8 illustre l'encapsulation, dans des billes de pectine, d'ADN plasmidique libre ou complexé à des lipides cationiques (Lipoplexe) ou à un polymère cationique (Polyplexe).

### Exemple 1: Préparation des formes galéniques

On introduit goutte à goutte une solution aqueuse de pectine à 6% (OF 400 ou OG 175C Unipectine^{®} de chez Degussa) dans une solution de chlorure de calcium à 6% (m/v). La solution de pectine est introduite dans la solution de chlorure de calcium grâce à une tubulure Tygon^{®} raccordée à une pompe péristaltique (Microperpex^{®} LKB Bromma) - La solution passe au travers d'une aiguille de 0,8 mm de diamètre (21G, Nedus Terumo) pour former des gouttes de pectine qui au contact du chlorure de calcium (40 ml) gélifient instantanément et donnent des billes de pectinate de calcium. Les billes sont maintenues dans le chlorure de calcium, sous agitation lente, pendant 20 minutes.
Les billes blanches ne contenant pas de principe actif (β-lactamases) sont obtenues en partant d'une solution de pectine amidée (OG 175C) ou non amidée (OF 400) à 6%. Pour la préparation des billes chargées, le principe actif (β-lactamases, pénicillinases de type A extraite de Bacillus cereus de chez Sigma) est mélangé à la solution de pectine dans un rapport de 3% (vₚₐ/v_{pectine}).
Les billes de pectinate de calcium ainsi obtenues sont ensuite récupérées par filtration, rincées à l'eau distillée, placées sur une boîte de Pétri et séchées à l'étuve à 37 °C pendant 2 heures.
Pour la réticulation à la polyéthylènimine les billes non séchées, récupérées de la solution de CaCl₂ par filtration, sont introduites dans une solution aqueuse de polyéthylènimine (PEI) à 1% et y sont maintenues pendant 20 min sous légère agitation.
Les billes préparées à partir de la pectine non amidée OF 400 contiennent de 1 à 2, 5 UI/billes et les billes préparées à partir de pectine amidée OG 175C contiennent de 1 à 5 UI/billes.

### Exemple 2: Stabilité des billes

### 1. Mode opératoire.

Les billes sont préparées selon l'exemple 1 avec ou sans étape de réticulation; la durée de réticulation dans la PEI est de 20 minutes dans des solutions de concentrations allant de 0,6 à 1%.
Les billes sont placées soit dans du tampon phosphate (PBS 0,01M, pH 7,4), soit dans des milieux simulant les milieux digestifs (gastrique et intestinal USP XXIV) et le temps de désagrégation est observé.

### 2. Résultats.

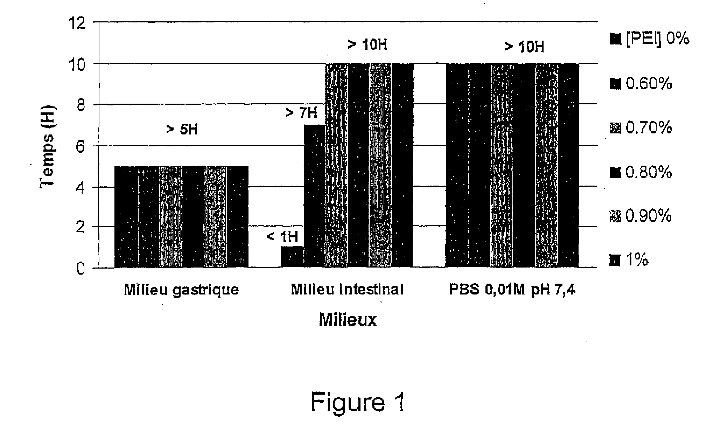

Ils sont donnés dans la figure 1.
Les billes réticulées ou non sont stables dans le PBS et dans le milieu gastrique. En revanche, les billes non réticulées sont instables dans le milieu intestinal, alors que les billes selon l'invention sont stables plus de 7 heures.

### Exemple 3: Caractéristiques morphologiques des billes.

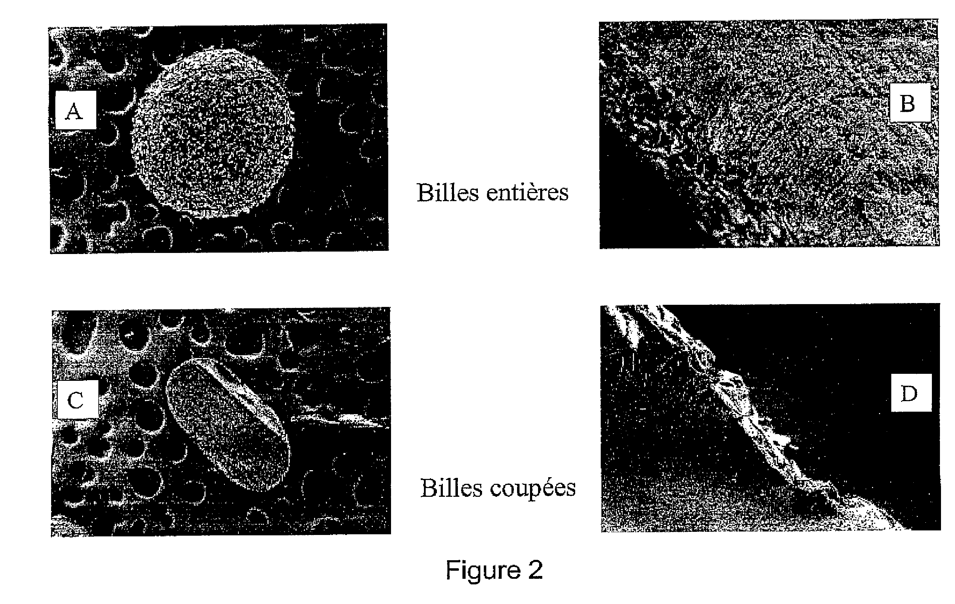

Elles sont illustrées dans les figures 2A à 2D.
On observe sur les coupes que le centre des billes est plein et dense. La coque en surface correspond à la PEI. L'intérieur et l'extérieur ont des structures différentes.

### Exemple 4: Cinétique de libération in vitro

### 1. Mode opératoire.

Des billes réticulées avec deux concentrations différentes en PEI (0,6 et 0,7%) sont préparées selon l'exemple 1 à partir de pectine amidée et contenant 5 UI/bille Elles sont laissées pendant 5 heures dans un milieu intestinal USP XXIV à pH 6,8, puis introduites en milieu colique synthétique à pH 6 enfermant des enzymes pectinolytiques (Pectinex^{®} Ultra SPL).
On mesure l'activité β-lactamase résiduelle dans les billes au cours du temps par spectrophotométrie en présence de nitrocéphine.

### 2. Résultats.

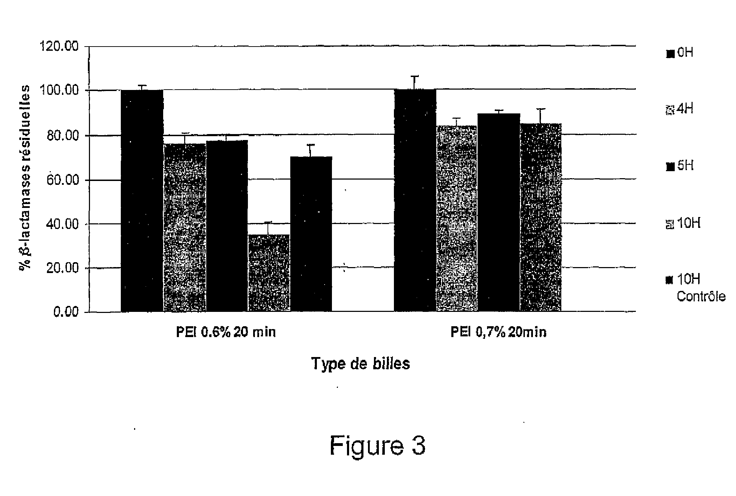

Ils sont illustrés dans la figure 3.
Après 5 heures d'incubation des billes en milieu intestinal (T_{5H}) moins de 25% de l'activité β-lactamases qu'elles contiennent sont libérés. La libération devient importante en milieu colique sous l'action des enzymes pectinolytiques (T_{10H}), pour les billes réticulées avec 0,6% de PEI, alors que le témoin sans enzymes pectinolytiques (T_{10H} contrôle) ne présente pas de modifications significatives d'activité β-lactamase. Par contre, les billes réticulées avec 0,7% de PEI ne voit pas leur activité diminuer après 5H en milieu colique. Ainsi la concentration en PEI modifie la résistance des billes et joue sur les temps de libération des principes actifs en milieu colique.

### Exemple 5: Cinétique de libération in vivo.

### 1. Mode opératoire.

Cet essai a été réalisé sur des souris mâles CD1. Les billes contiennent 4 UI/bille.
On administre per os aux souris des gélules contenant 10 billes. On récupère les selles aux temps 0, 2H, 3H, 4H, 5H, 6H, 7H et 8H et on réalise le dosage des β-lactamases dans ces selles (essai conduit sur 5 animaux pour chaque temps). De plus, on sacrifie aux temps 30 min, 2H et 4H une souris afin de récupérer les billes dans son tube digestif et d'observer leurs modifications morphologiques en microscopie électronique à balayage.

### 2. Résultats.

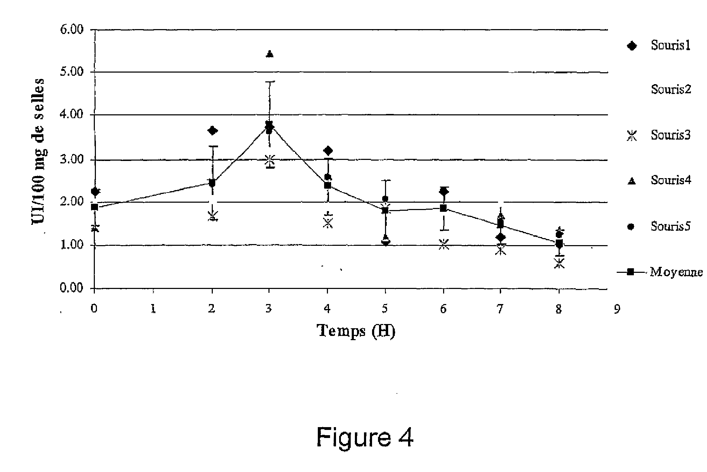

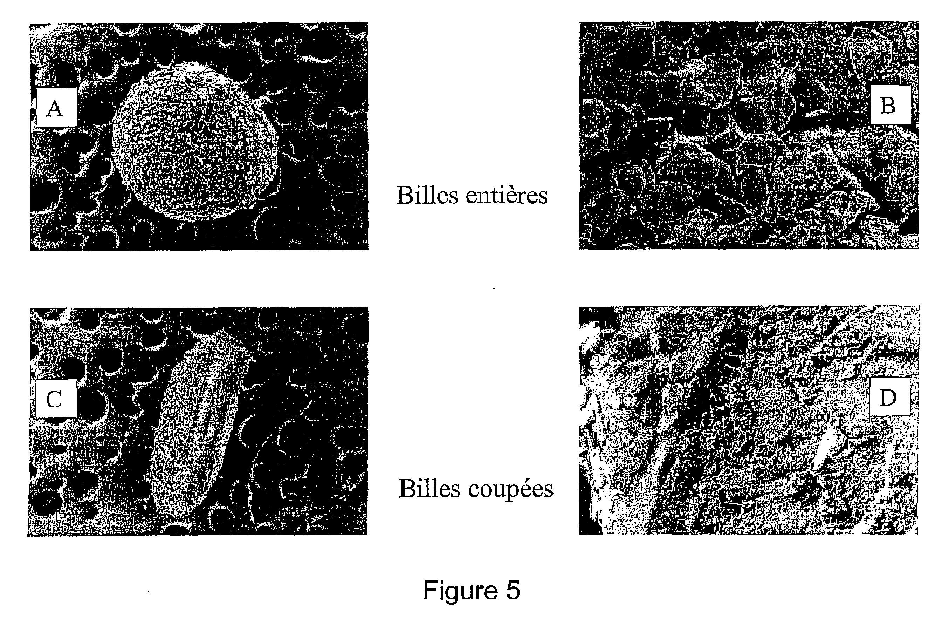

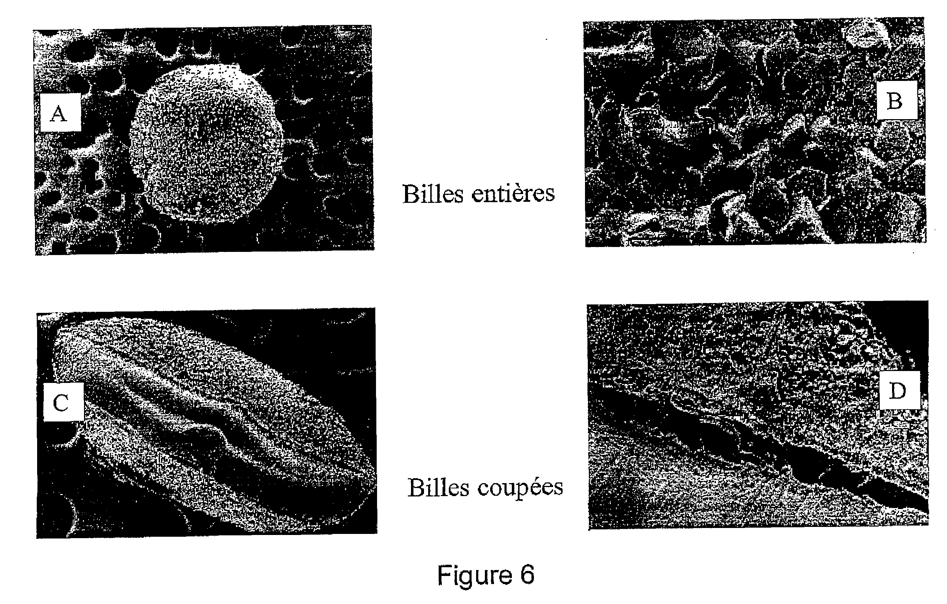

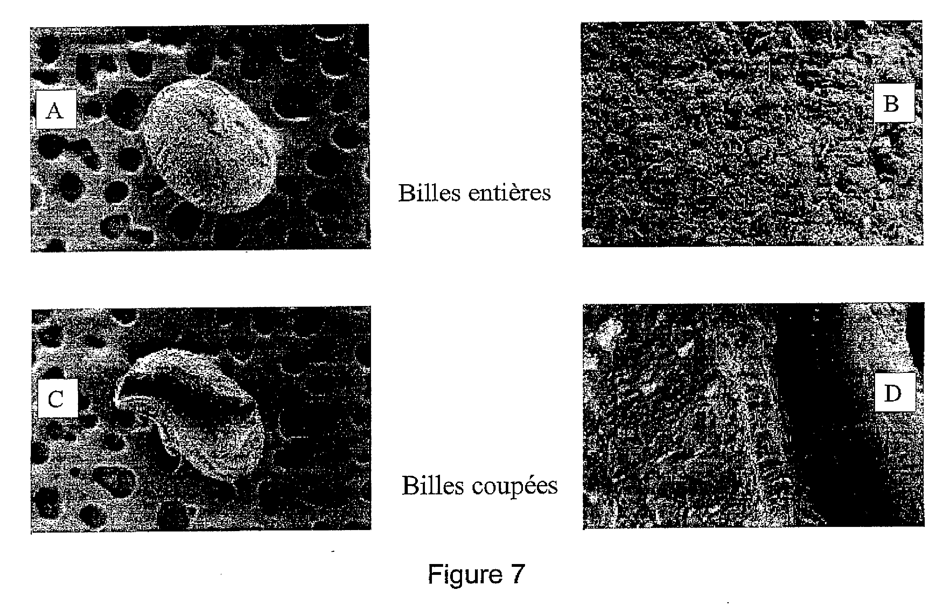

Ils sont illustrés dans les figures 4 à 7.
Les billes arrivent intactes dans le colon après environ 3 heures de transit.
Le taux de (β-lactamases libérées directement dans les selles de souris recueillies à différents temps après l'absorption des billes par voie orale montre que l'activité β-lactamases de base est faible au départ. 2 à 4 heures après l'administration, il y a une augmentation nette de cette activité, ce qui correspond bien au transit des billes (figure 4).
Les photos prises en microscopie électronique à balayage montrent l'intégrité de la bille dans les différents endroits du tube digestif.
La structure est légèrement fragilisée au niveau de l'intestin grêle et l'intérieur est complètement détruit au niveau colique où les billes apparaissent porteuses d'une cavité.
Les particules, illustrées dans la figure 5, ayant séjourné dans l'estomac ont un aspect très proche de celles n'ayant subi aucun traitement (figure 2). En effet la surface a le même aspect rugueux et irrégulier (figures 5A et 5B), dû à la présence de polyéthylenimine, et la section des billes apparaît uniforme et dense (figures 5C et 5D).
Au bout de 2 h, on voit apparaître une légère déformation des billes (figure 6A) mais les particules ont toujours le même aspect en surface (figure 6B) et une section dense (figure 6C) bien qu'un peu fragilisée par le séjour dans l'intestin grêle (figure 6D).
En fin de transit, c'est-à-dire 4 h après l'administration, les billes se retrouvent dans le colon; l'aspect externe des particules est inchangé (figure 7A) avec les mêmes irrégularités de surface dues à la polyéthylenimine (figure 7B). En revanche, la section des billes est creuse (figure 7C et 7D), du fait de la dégradation du réseau central de pectinate de calcium par les enzymes pectinolytiques coliques. Au final, il ne reste que la « *coque* » externe formée par la polyéthylenimine.

### Exemple 6: Encapsulation d'érythromycine estérase

### 6.1 Production d'une fraction soluble contenant l'érythromycine estérase

### 6.1.1. Mode opératoire

La culture est réalisée à partir de la souche d'*E*. *coli* C600 pIP1100 de l'Institut Pasteur. Les conditions de culture sont les suivantes : ensemencement du milieu Mueller-Hinton à 0,5% à partir d'une préculture d'environ 20h, volumes de culture de 200 ou 400 mL en erlenmeyer, agitation fixée à 150 rpm, température de 37°C.
Un test de GOTS a permis d'établir que la souche produisait bien de l'érythromycine estérase.
3,6L de culture d'*E*. *coli* C600 pIP1100 ont été concentrés selon le protocole suivant :
- Centrifugation pendant 15 min à 3 400 g
- Reprise du culot dans du tampon phosphate de potassium 5mM, pH 7,5, volume final 70 mL
- 2^{ème} centrifugation de surnageant 15 min à 3 400 g
- Reprise du culot dans 20 mL de tampon phosphate de potassium 5mM, pH 7,5
- Rassemblement des culots des 2 centrifugations (environ 100 mal)
- Lavage des culots et centrifugation (10 min à 12 000 g)
- 2^{ème} centrifugation du surnageant (10 min 12 000 g)
- Volume final des culots repris dans le tampon phosphate de potassium : 100 mL
L'érythromycine estérase est une enzyme intracellulaire. C'est pourquoi sa solubilisation nécessite le cassage des cellules. Cette opération a été effectuée par bonification des culots de centrifugation repris dans le tampon phosphate de potassium 5mM, pH 7,5 selon le protocole décrit ci-dessous.
- Ajout de 1% Triton X100 (v/v)
- Refroidissement à 5°C
- Phonolyse 7 cycles de 1 min, température initiale 5°C, température maximale 15°C, puissante 100% (500W, 20kHz) ; température ramenée à 5°C après chaque cycle
- Centrifugation 10 min à 12 000 g
- Reprise du culot dans 10 mL de tampon phosphate de potassium 5mM, pH 7,5
- Récupération et congélation du surnageant (91 mL) = solution A
L'activité érythromycine estérase a été évaluée par le dosage microbiologique dans le surnageant et dans les insolubles (débris cellulaires) selon les techniques connues de l'homme du métier.

### 6.1.2. Résultats

Les résultats sont présentés dans le Tableau 2.

**Tableau 2**

| Echantillon | Diamètre d'inhibition (mm) | | | |
|---|---|---|---|---|
| | T0 | T30 | T60 | T120 |
| Surnageant après sonification | 31 | 25 | 18 | - |
| | 21 | - | 18 | 14 |
| Culot après sonification | 30 | 28 | 24 | - |
| | 22,5 | - | 19,5 | 19 |

L'activité érythromycine estérase est évaluée à partir du diamètre d'inhibition.
Celle-ci est de 2 U/mL pour le surnageant de phonolyse et 1,5 U/mL pour le culot de phonolyse (1 Unité (U) = 1 µg d'érythromycine dégradée par min).
Le bilan de récupération de l'activité érythromycine estérase est présenté dans le Tableau 3 ci-dessous.

**Tableau 3**

| Echantillon | Activité estimée (U/mL) | Volume (mL) | Activité totale estimée (U) |
|---|---|---|---|
| Surnageant après sonification | 2,0 | 92 | 184 |
| Culot après sonification | 1,5 | 10 | 15 |

Les résultats montrent clairement que l'essentiel de l'activité érythromycine estérase présente a bien été solubilisée dans le milieu de phonolyse.

### 6.2 Encapsulation d'érythromycine estérase

### 6.2.1. Mode opératoire

L'encapsulation est réalisée à partir de la fraction soluble non purifiée obtenue après cassage des cellules (solution A) selon le protocole suivant.
- Solubilisation de 0,5 g de pectine dans les 10 mL de solution A afin d'obtenir une concentration finale de pectine de 5% (solution B). La pectine est ajoutée très progressivement sous agitation magnétique afin de ne pas entraîner de trop brusques variations de pH. Le pH est maintenu aux environs de 7 par addition de quelques gouttes de soude 1M.
- Dispersion de la solution de pectine (solution B) goutte à goutte à l'aide d'une pompe péristaltique dans 40 mL de CaCl₂ à 6%. Les billes ainsi formées sont maintenues dans le CaCl₂ pendant 20 min, récupérées par filtration sur Büchner puis rincées à l'eau déminéralisée.
- Réticulation des billes par bain dans une solution de PEI à 0,6% pendant 20 min sous agitation magnétique.
- Récupération des billes réticulées par filtration.
- Séchage des billes à température ambiante (20°C). 567 billes ont été préparées au total avec 6,1 mL de mélange pectine/solution A, pour une activité de 12,2 U.
- Désagrégation des billes séchées dans un tampon HEPES/NaCl/EDTA 1%.

### 6.2.2. Résultats

L'activité érythromycine estérase présente dans la solution initiale de pectine et celle libérée dans le milieu de désagrégation sont dosées selon le même protocole que précédemment.
Les résultats du dosage microbiologique sont présentés dans les Tableaux 4 et 5.

**Tableau 4**

| Echantillon | Diamètre moyen d'inhibition (mm) |
|---|---|
| Pectine / Solution A (solution B)-T0 | 23 |
| Pectine / Solution A -T3h | 19 |
| Billes désagrégées-T0 | 24 |
| Billes désagrégées-T3h | 18 |

**Tableau 5**

| Echantillon | Activité estimée (U) |
|---|---|
| Pectine (Solution B) | 2,4 |
| Billes désagrégées | 2,2 |

Les résultats montrent que l'activité mesurée en présence de pectine (solution B) est de 2,4 U, alors que l'activité théorique présente devrait être d'environ 12 U (6,1 mL à 2 U/mL, d'après le dosage d'érythromycine estérase dans le surnageant de phonolyse (Tableau 3). Le dosage de l'activité enzymatique de billes après désagrégation a été estimée à 2,2 U ; elle représente 90% de l'activité initiale introduite dans les billes.
Ces résultats permettent d'établir sans ambiguïté la présence d'activité érythromycine estérase dans la fraction finale après encapsulation de l'enzyme et désagrégation des billes.
Ces résultats permettent d'établir sans ambiguïté la présence d'activité érythromycine estérase dans la fraction finale après encapsulation de l'enzyme et désagrégation des billes.

### Exemple 7: Encapsulation d'ADN dans les billes de pectinate de calcium

### 7.1 Préparation de l'ADN

Le principe actif encapsulé ici est un plasmide radiomarqué au Phosphore 33. Le radiomarquage se fait à l'aide du « Nick Translation Kit » N5500 de chez Amersham Biosciences selon le protocole décrit par le fournisseur.

### 7.2 Encapsulation

### 7.2.1. Mode opératoire

L'ADN encapsulé est soit sous forme libre, soit complexé à des lipides cationiques (Lipoplexe) ou à un polymère cationique (Polyplexe) selon le mode opératoire décrite dans l'exemple 1.
Pour l'ADN libre, environ 5 ug d'ADN radiomarqué en solution dans 750 µL d'eau MilliO sont introduits dans 0,75g d'une solution de pectine, amidée ou non, à 10% afin d'obtenir une concentration finale en pectine de 5%. Dans le cas des lipoplexes, 375 µL d'une solution aqueuse d'ADN radiomarqué sont mélangés à 375 µL d'une suspension de liposomes cationiques (ratio N/P de 10). Les 750 µL de lipoplexes ainsi obtenus sont ensuite mélangés à 0,75 g de solution de pectine à 10% afin d'obtenir une concentration finale en pectine de 5%.
Dans le cas des polyplexes, 375 µL d'une solution aqueuse d'ADN radiomarqué sont mélangés à 375 µL d'une solution aqueuse de PEI 4mM. 375 µL de la suspension de polyplexes ainsi obtenus sont ensuite mélangés à 0,75 g de solution pectine à 10% afin d'obtenir une concentration finale en pectine de 5%.
Les billes de pectinate de calcium encapsulant l'ADN libre ou complexé sont ensuite préparées à partir des solutions obtenues ci-dessus selon la méthode décrite dans l'exemple 1. La concentration en chlorure de calcium utilisée ici est de 5% et celle de PEI pour la réticulation est de 0,6%.

### 7.2.2. Résultats

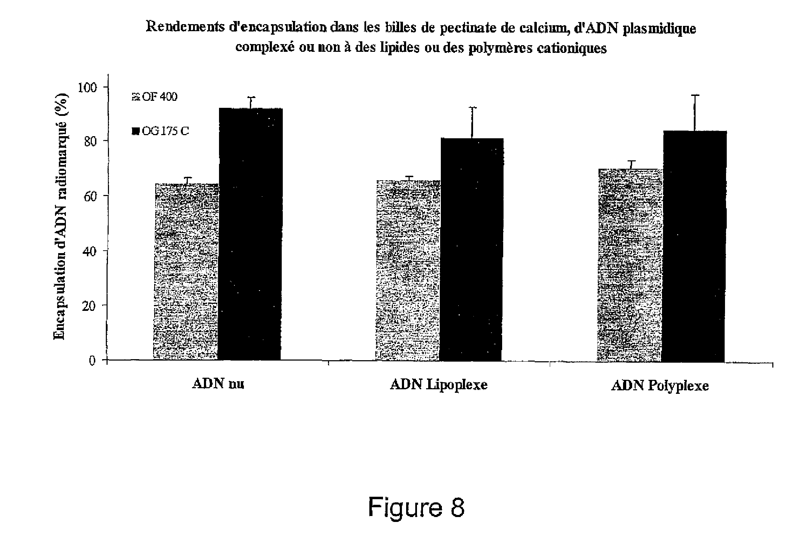

Ils sont illustrés à la figure 8 qui montre les rendements d'encapsulation d'un ADN plasmidique dans des billes de pectine amidée ou non amidée.
L'ADN encapsulé est soit sous forme libre, soit complexé à des lipides cationiques (Lipoplexe) ou à un polymère cationique (Polyplexe).
Les rendements d'encapsulation en ADN varient entre 60 et 90% selon le type de pectine utilisée. Ils sont généralement plus importants avec la pectine amidée. La complexation à des lipides ou à un polymère cationique n'entraîne pas de modifications significatives de ces rendements qui restent relativement élevés.
L'invention concerne donc les items suivants:
1. Formes galéniques multiparticulaires aptes à être utilisées par voie orale et destinées à la délivrance colique de principes actifs choisis dans le groupe comprenant les enzymes capables d'inactiver les macrolides et apparentés et les enzymes capables d'inactiver les quinolones.
2. Formes galéniques multiparticulaires selon l'item 1, caractérisées en ce que l'enzyme capable d'inactiver les macrolides est l'érythromycine estérase.
3. Formes galéniques multiparticulaires caractérisées en ce qu'elles sont aptes à être utilisées par voie orale et destinées à la délivrance colique de principes actifs comprenant des billes de pectine présente sous forme d'un sel cationique renfermant le principe actif, ladite pectine étant réticulée par un polymère cationique.
4. Forme galénique selon l'item 3, caractérisée en ce que le polymère cationique est choisi dans le groupe constitué par la polyéthylènimine, la polylysine, le chitosan et leurs dérivés.
5. Formes galéniques selon l'un quelconque des items 3 à 4, caractérisées en ce que le polymère cationique a un poids moléculaire compris entre 10 000 et 100 000 Daltons, de préférence entre 20 000 et 50 000 Daltons.
6. Formes galéniques selon l'un quelconque des items 3 à 5, caractérisées en ce que le sel de pectine est un pectinate de calcium préparé à partir de pectine amidée ou non amidée.
7. Formes galéniques selon l'un quelconque des items 3 à 6, caractérisées en ce qu'elles sont préparées à partir d'une solution de pectine à 4-10% (m/v), avantageusement de 4 à 7% (m/v), d'une solution de chlorure de calcium à 2-10% (m/v) et d'une solution de 10 polyéthylènimine à 0,5-2% (m/v).
8. Formes galéniques selon l'un quelconque des items 3 à 7, caractérisées en ce que la solution de pectine est à 6%, la solution de chlorure de calcium à 6% et la solution de polyéthylènimine à 1% ou 0,6%.
9. Formes galéniques selon l'un quelconque des items 3 à 8, caractérisées en ce que le principe actif est choisi parmi les antibiotiques, les composés anti-inflammatoires, anti-histaminiques, anti-cholinergiques, antiviraux, antimitotiques, les peptides, les protéines, les gènes, les oligonucléotides anti-sens, les agents de diagnostic, les agents immunosuppressifs et/ou les bactéries, de préférence parmi les agents antitumoraux et les enzymes capables d'inactiver les antibiotiques au niveau du colon.
10. Formes galéniques selon l'item 9, caractérisées en ce que les enzymes sont choisis dans le groupe constitué par les β-lactamases, les enzymes capables d'inactiver les macrolides et apparentés, de préférence l'érytrhomycine estérase et les enzymes capables d'inactiver les quinolones.
11. Procédé de préparation d'une forme galénique selon l'un quelconque des items 3 à 8, caractérisé en ce que l'on ajoute une solution aqueuse de pectine contenant le principe actif à une solution aqueuse d'un sel cationique divalent, de manière à obtenir des billes de pectine sous forme d'un sel cationique renfermant le principe actif, et que l'on réticule lesdites billes par introduction dans une solution aqueuse du polymère cationique.

## Revendications

1. Forme galénique multiparticulaire apte à être utilisée par voie orale et destinée à la délivrance colique de principes actifs **caractérisée en ce qu'**elle comprend un principe actif choisi dans le groupe comprenant les enzymes capables d'inactiver les antibiotiques.

2. Forme galénique multiparticulaire selon la revendication 1, **caractérisée en ce que** les enzymes sont choisies dans le groupe comprenant les β-lactamases, les enzymes capables d'inactiver les macrolides et apparentés et les enzymes capables d'inactiver les quinolones.

3. Forme galénique multiparticulaire selon la revendication 2, **caractérisée en ce que** l'enzyme capable d'inactiver les macrolides est l'érythromycine estérase.

4. Forme galénique multiparticulaire selon l'une quelconque des revendications 1 à 3, **caractérisée en ce qu'**elle comprend des billes de pectine présente sous forme d'un sel cationique renfermant le principe actif, ladite pectine étant réticulée par un polymère cationique.

5. Forme galénique selon la revendication 4, **caractérisée en ce que** le polymère cationique a un poids moléculaire compris entre 10 000 et 100 000 Daltons.

6. Forme galénique selon la revendication 5, **caractérisée en ce que** le polymère cationique a un poids moléculaire compris entre 20 000 et 50 000 Daltons.

7. Forme galénique selon l'une quelconque des revendications 4 à 6, **caractérisée en ce que** le sel de pectine est un pectinate de calcium.

8. Forme galénique selon l'une quelconque des revendications 4 à 7, **caractérisée en ce que** la pectine est méthylée ou non méthylée, amidée ou non amidée,

9. Forme galénique selon l'une quelconque des revendications 1 à 8, **caractérisée en ce qu'**elle est sous forme de capsule ou gélule.

10. Procédé de préparation d'une forme galénique selon l'une quelconque des revendications 1 à 9, **caractérisé en ce qu'**il comprend les étapes suivantes:
a) préparation d'une solution aqueuse de pectine contenant le principe actif,
b) addition de la solution obtenue à l'étape a) à une solution aqueuse d'un sel cationique divalent, de manière à obtenir des billes de pectine sous forme d'un sel cationique renfermant le principe actif, et
c) réticulation desdites billes par introduction dans une solution aqueuse du polymère cationique.

11. Procédé de préparation d'une forme galénique selon la revendication 10, **caractérisé en ce qu'**il comprend les étapes suivantes:
a) préparation d'une solution de pectine à 4-10% (m/v), avantageusement de 4 à 7% (m/v), contenant le principe actifs,
b) addition de la solution obtenue à l'étape a) à une solution de chlorure de calcium à 2-10% (m/v) et
c) réticulation par une solution de polymère cationique à 0,5-2%(m/v).

12. Forme galénique multiparticulaire apte à être utilisée par voie orale et destinée à la délivrance colique de principes actifs **caractérisée en ce qu'**elle est susceptible d'être obtenue par un procédé selon la revendication 10 ou 11.

13. Composition pharmaceutique comprenant une forme galénique selon l'une quelconque des revendications 1 à 9 , e t 12.

14. Composition pharmaceutique selon la revendication 13, **caractérisée en ce qu'**elle est administrée en combinaison avec l'antibiotique correspondant pour une utilisation simultanée ou successive en thérapie antibiotique.

15. Composition pharmaceutique selon la revendication 13 ou 14, **caractérisée en ce qu'**elle est sous forme de gélule ou capsule.
